(19) Europäisches Patentamt
European Patent Office
Office européen des brevets

(11) **EP 1 757 216 A1**

(12) **EUROPEAN PATENT APPLICATION**
published in accordance with Art. 158(3) EPC

(43) Date of publication:
**28.02.2007 Bulletin 2007/09**

(51) Int Cl.:
**A61B 1/00** (2006.01)

(21) Application number: **05743317.9**

(22) Date of filing: **24.05.2005**

(86) International application number:
**PCT/JP2005/009420**

(87) International publication number:
**WO 2005/115220 (08.12.2005 Gazette 2005/49)**

(84) Designated Contracting States:
**DE FR GB**

(30) Priority: **27.05.2004 JP 2004157391**
**27.05.2004 JP 2004157392**

(71) Applicant: **MATSUSHITA ELECTRIC INDUSTRIAL CO., LTD.**
**Osaka 571-8501 (JP)**

(72) Inventors:
• **OKADA, Eiji**
**c/o Matsushita Electric Industrial Co.**
**1-3-7 Shiromi, Chuo-ku, Osaka 540-6319 (JP)**

• **ASAKAWA, Y.**
**c/o Matsushita Electric Industrial Co.**
**1-3-7 Shiromi, Chuo-ku, Osaka 540-6319 (JP)**

(74) Representative: **Grünecker, Kinkeldey,**
**Stockmair & Schwanhäusser**
**Anwaltssozietät**
**Maximilianstrasse 58**
**80538 München (DE)**

(54) **TRANSMITTER, RECEIVER, AND TRANSMISSION/RECEPTION SYSTEM**

(57) A transmission/reception system is adaptable to be used for an encapsulated endoscope that can be swallowed by a living organism. The system includes a transmitter and a receiver. The transmitter includes a voltage converter for generating a voltage according to transmission data, a voltage-controlled oscillator for generating a signal of a frequency corresponding to the voltage generated by the voltage converter under non-feedback control, and a first antenna for emitting the signal generated by the voltage-controlled oscillator. The a receiver includes a second antenna for receiving the signal emitted from the first antenna, a first amplifier for amplifying the signal received by the second antenna, an oscillator for generating a local oscillation signal, a mixer for mixing the signal amplified by the first amplifier with the local oscillation signal and converting the signal amplified by the first amplifier into an intermediate-frequency (IF) signal, a detector for detecting the IF signal, and a controller for changing a frequency of the local oscillation signal according to the frequency of the received signal, so that the IF signal has a predetermined frequency. The transmitter of this transmission/reception system has a small size and consumes small power.

Fig. 1B

## Description

TECHNICAL FIELD

The present invention relates to a transmission/reception system including a small transmitter and a receiver corresponding to the transmitter for use in an encapsulated endoscope.

BACKGROUND ART

An encapsulated endoscope including a transmitter is swallowed for inspection, thus providing less physical burden on a subject and allowing the inspection to be executed regardless of time. Hence, the encapsulated endoscope is used for inspection of small intestines, which can be hardly inspected.

International Patent Application Publication No.WO01/65995 discloses a conventional transmission/ reception system including a conventional transmitter and receiver.

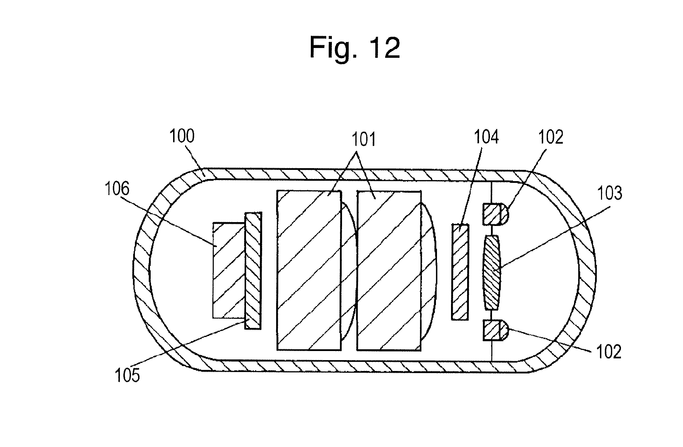

Fig. 12 is a sectional view of a conventional encapsulated endoscope including conventional transmitter 105. Light emitting diode (LED) 102 illuminates an inside of a human organ, such as an esophagus or bowel. Optical system 103 and image-capturing device 104 captures an image of a portion which is illuminated. Transmitter 105 and antenna 106 transmit image data of the image. Capsule 100 accommodates the above components and batteries 101, a power source of them, therein.

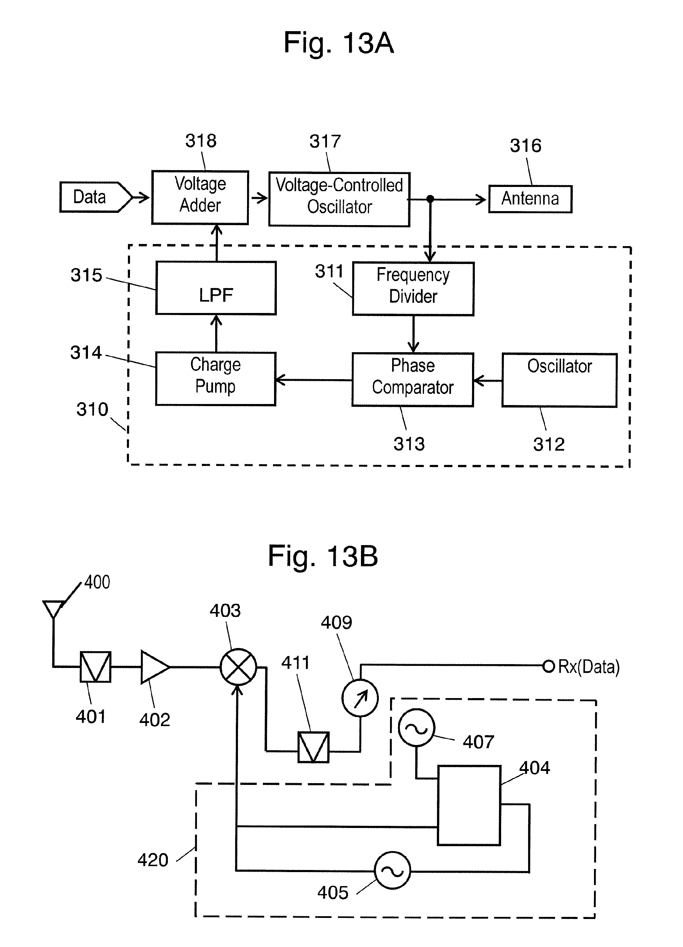

Fig. 13A is a block diagram of transmitter 105. Voltage-controlled oscillator 317 outputs a signal having a frequency determined according to an input voltage. Frequency divider 311 converts the frequency of the signal into a low frequency, 1/N times of the frequency, and outputs the converted signal. Phase comparator 313 measures a phase difference between the signal supplied from frequency divider 311 and a signal supplied from reference oscillator 312. Charge pump 314 outputs a voltage corresponding to the phase difference. Loop filter 315 smoothes the voltage supplied from charge pump 314 and supplies the smoothed voltage into voltage-controlled oscillator 317. Thus, voltage-controlled oscillator 317 outputs a signal having a frequency N times the oscillating frequency of reference oscillator 312.

Voltage adder 318 Inserted between loop filter 315 and voltage-controlled oscillator 317. Voltage adder 318 adds a voltage which corresponds to digital data "0" and "1" of transmission data (image data) into a voltage supplied form loop filter 315, and inputs the data into voltage-controlled oscillator 317. Thus, a modulated wave that is frequency-modulated corresponding to data "0" and "1" is output from antenna 316.

Fig. 13B is a block diagram of a receiver corresponding to the conventional transmitter shown in Fig. 13A. The modulated wave input into antenna 400 has radio waves of unnecessary frequencies removed therefrom by band-pass filter 401, is amplified by low-noise amplifier 402, and is supplied to mixer 403. Mixer 403 has a local oscillation signal input thereto. The local oscillation signal is generated by frequency synthesizer 420 including reference signal oscillator 407, voltage-controlled oscillator 405, and phase-locked loop (PLL) circuit 404, in other words, generated by voltage-controlled oscillator 405. PLL circuit 404 compares the phase of a reference signal generated by reference signal oscillator 407 and the phase of a signal generated by voltage-controlled oscillator 405, and inputs a voltage corresponding to the phase difference into voltage-controlled oscillator 405. PLL circuit 404 may include a frequency divider for dividing the frequency of the reference signal from reference signal oscillator 407 and a frequency divider for dividing the frequency of the signal generated by voltage-controlled oscillator 405, according to the frequencies to be compared. Mixer 403 mixes a signal supplied from low-noise amplifier 402 with a local oscillation signal to generate an intermediate-frequency (IF) signal. The IF signal has its thermal noises of unnecessary frequencies suppressed by band-pass filter 411, is demodulated by detector 409, and is converted into a voltage corresponding to data "0" and "1".

In transmitter 105, phase-locked loop (PLL) circuit 310 including frequency divider 311, phase comparator 313, charge pump 314, and loop filter 315 stabilizes a center frequency of the modulated wave.

The frequency band necessary for a receiver outside of a human body is substantially equal to the bandwidth occupied by the modulated wave that is determined by the transmission speed of the transmission data. The occupied bandwidth is limited by the filter as to suppress the unnecessary thermal noise, accordingly allowing the receiver to receive the modulated wave at high sensitivity.

Conventional transmitter 105 includes PLL circuit 310 of large circuitry and stabilizes the frequency of the modulated wave to be transmitted. The PLL circuit accordingly simplifies the structure of the receiver, but causes transmitter 105 to have a large size and consume large power. Such large size of the transmitter may cause capsule 100 to be too large to be swallowed. Such large power consumption may cause batteries 101 to run down before the endoscope reaches the portion to be inspected. In order to be prevented from running down, batteries 101 necessarily have large capacities, and have large sizes accordingly.

SUMMARY OF THE INVENTION

A transmission/reception system is adaptable to be used for an encapsulated endoscope that can be swallowed by a living organism. The system includes a transmitter and a receiver. The transmitter includes a voltage converter for generating a voltage according to transmission data, a voltage-controlled oscillator for generating a signal of a frequency corresponding to the voltage generated by the voltage converter under non-feedback control, and a first antenna for emitting the signal generated by the voltage-controlled oscillator. The a receiver includes a second antenna for receiving the signal emitted from the first antenna, a first amplifier for amplifying the signal received by the second antenna, an oscillator for generating a local oscillation signal, a mixer for mixing the signal amplified by the first amplifier with the local oscillation signal and converting the signal amplified by the first amplifier into an intermediate-frequency (IF) signal, a detector for detecting the IF signal, and a controller for changing a frequency of the local oscillation signal according to the frequency of the received signal, so that the IF signal has a predetermined frequency.

The transmitter of this transmission/reception system has a small size and consumes small power.

BRIEF DESCRIPTION OF THE DRAWINGS

Fig. 1A is a block diagram of a transmitter of a transmission/reception system in accordance with Exemplary Embodiment 1 of the present invention.
Fig. 1B is a block diagram of a receiver of the transmission/reception system in accordance with Embodiment 1.
Fig. 1C is a block diagram of a phase-locked loop circuit in the receiver in accordance with Embodiment 1.
Fig. 2 is a sectional view of the transmitter in a capsule in accordance with Embodiment 1.
Fig. 3 is a perspective view of an antenna of the transmitter in accordance with Embodiment 1.
Fig. 4 is a circuit diagram of a voltage converter of the transmitter in accordance with Embodiment 1.
Fig. 5 is a circuit diagram of an LC resonator of the transmitter in accordance with Embodiment 1.
Fig. 6 is a block diagram of a power supply and the transmitter in accordance with Embodiment 1.
Fig. 7 shows a circuit pattern of the transmitter in accordance with Embodiment 1.
Fig. 8A is a block diagram of another transmitter in accordance with Embodiment 1.
Fig. 8B is a block diagram of still another transmitter in accordance with Embodiment 1.
Fig. 9 is a block diagram of a further transmitter in accordance with Embodiment 1.
Fig. 10 is a block diagram of a receiver in accordance with Exemplary Embodiment 2 of the invention.
Fig. 11 is a block diagram of a receiver in accordance with Exemplary Embodiment 3 of the invention.
Fig. 12 is a sectional view of a conventional encapsulated endoscope.
Fig. 13A is a block diagram of a conventional transmitter.
Fig. 13B is a block diagram of a conventional receiver.

REFERENCE NUMERALS

| | |
|---|---|
| 10 | Image-Capturing Circuit (Transmission Data Generator) |
| 12 | Voltage Converter |
| 13 | Voltage-Controlled Oscillator |
| 15 | Resonator |
| 16 | Antenna |
| 51A | Resistor (First Resistor) |
| 51B | Resistor (Second Resistor) |
| 52 | Capacitor |
| 200 | Antenna |
| 200A | Antenna |
| 201 | Band-Pass Filter |
| 202 | Low-Noise Amplifier (First Amplifier) |
| 202A | Low-Noise Amplifier (Second Amplifier) |
| 203 | Mixer |
| 204 | Phase-Locked Loop (PLL) Circuit |
| 205 | Voltage-Controlled Oscillator |
| 207 | Reference Signal Oscillator |
| 208 | IF Amplifier |
| 209 | Detector |
| 210 | Controller |
| 211 | Band-Pass Filter |
| 212 | Switch |
| 213 | Switch (First Switch) |
| 216 | Switch (Second Switch) |
| 1201 | Frequency Divider (First Frequency Divider) |
| 1202 | Frequency Divider (Second Frequency Divider) |
| 1203 | Phase Comparator |
| 1204 | Charge Pump |

DETAILED DESCRIPTION OF THE PREFERRED EMBODIMENTS EXEMPRALY EMBODIMENT 1

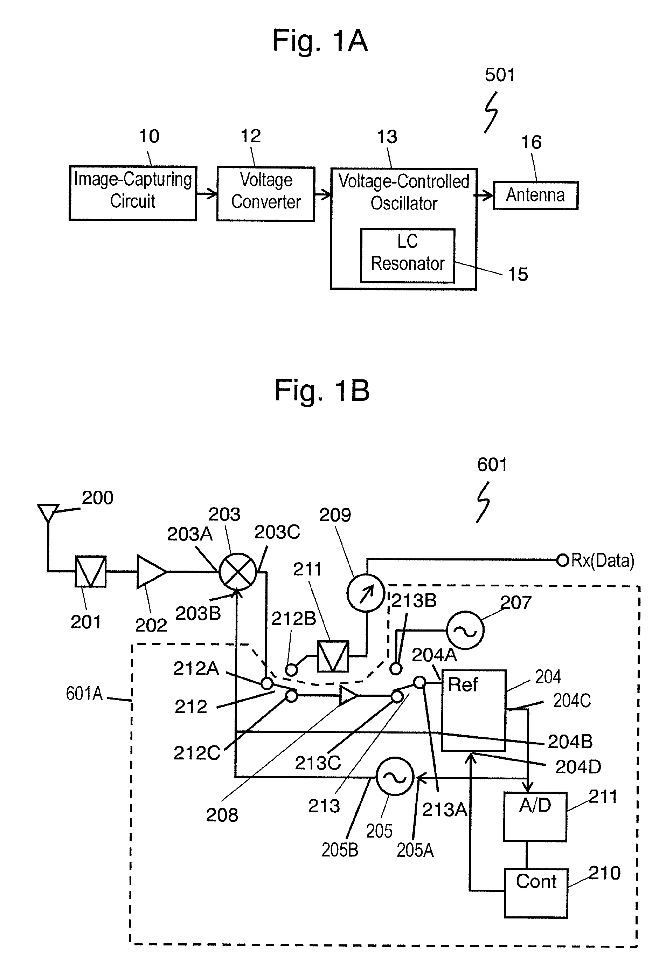

Fig. 1A is a block diagram of transmitter 501 of a transmission/reception system in accordance with Exemplary Embodiment 1 of the present invention. Image-capturing circuit 10, a transmission data generator, captures an image, and outputs image data, i.e., transmission data, having digital data "0" and "1" as a voltage or a current. Voltage converter 12 converts the image data supplied form image-capturing circuit 10 into a predetermined voltage. Voltage-controlled oscillator 13 generates a high-frequency signal of a frequency determined according to the voltage. The high-frequency signal is frequency-shift-keying-modulated with the image data, and is emitted through antenna 16 as a modulated wave. That is, voltage-controlled oscillator 13 of transmitter 501 generates signals under non-feedback control, while a conventional transmitter shown in Fig. 13 generates signals under feedback control.

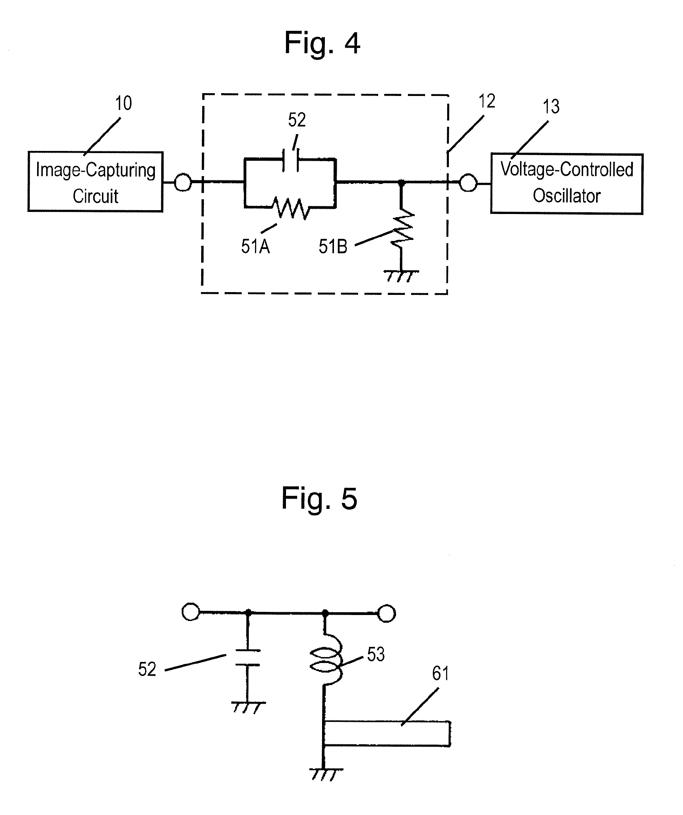

Fig. 4 is a circuit diagram of voltage converter 12 of transmitter 501. Voltage converter 12 includes resistor 51A connected to image-capturing circuit 10, resistor 51B connected in series with resistor 51A and grounded, and capacitor 52 connected in parallel with resistor 51A, thus providing a resistor divider circuit. Voltage converter 12 converts an output of the image-capturing circuit into a predetermined voltage. Capacitor 52 suppresses a rounding generated at a voltage change and shapes the waveform of the output.

Voltage-controlled oscillator 13 includes resonator 15, a variable capacitor, a transistor, an inductor, a capacitor, and a resistor. Fig. 5 is a circuit diagram of resonator 15. Resonator 15 is an LC resonator including capacitor 52 and chip inductor 53. Circuit pattern 53A is connected to chip inductor 53. Trimming part 61 made of an electrically-conductive pattern is connected to circuit pattern 53. The total inductance of trimming part 61 and chip inductor 53 serve as an inductor of resonator 15. Trimming part 61 made of the electrically-conductive pattern is trimmed with laser or other means as to change the inductance of resonator 15, accordingly adjusting the output frequency of voltage-controlled oscillator 13 precisely. Capacitor 52 may be a temperature-compensation capacitor to decrease variations in the capacitance of the capacitor to temperature changes, thus decreasing variations in the output frequency of oscillator 13 to temperature changes.

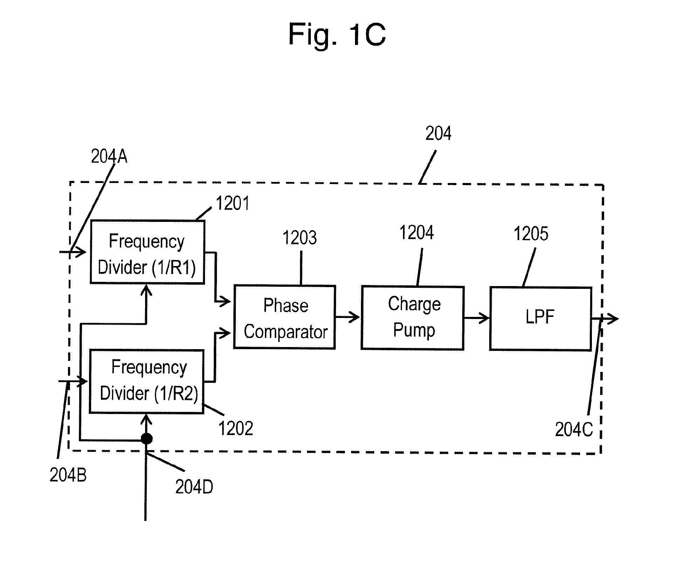

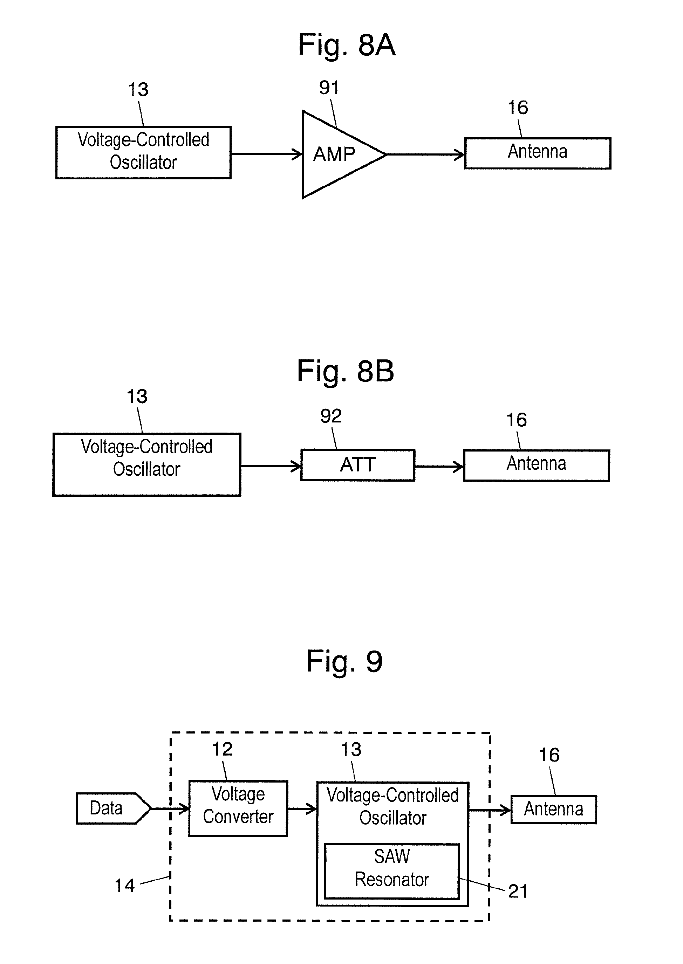

Fig. 9 is a block diagram of another transmitter 501A in accordance with Embodiment 1. Voltage-controlled oscillator 13 includes surface acoustic wave (SAW) resonator 21 instead of LC resonator 15 shown in Figs. 1 and 5. SAW resonator 21 can selects frequencies more sharply than LC resonator 15, accordingly stabilizing the frequency of the signal supplied form voltage-controlled oscillator 13.

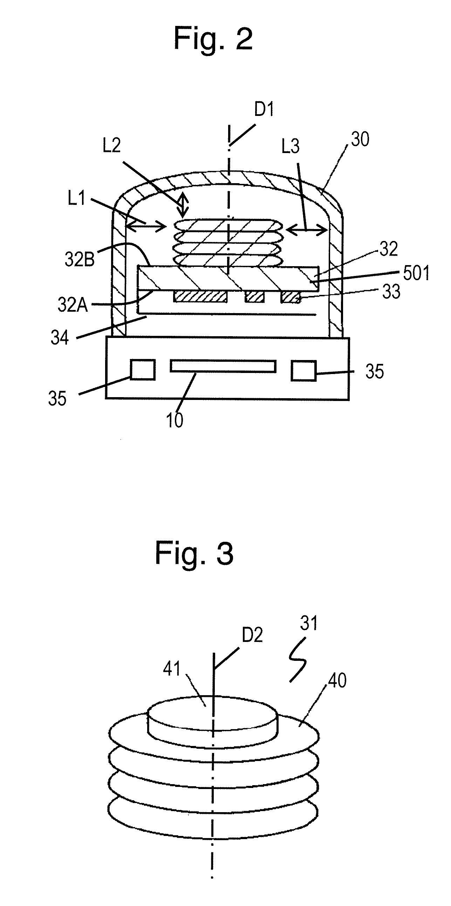

Fig. 2 is a sectional view of capsule 30 for accommodating transmitter 501 therein. Capsule 30 has a size to be swallowed by a living organism, such as a person to be inspected. Light-emitting diode (LED) 35 illuminates a portion in the living organism to have an image of the portion of be captured with image-capturing circuit 10. Component 33 mounted on one surface 32A of board 32 constitutes transmitter 501 shown in Fig. 1A. Helical antenna 31 functioning as antenna 16 shown in Fig. 1A is mounted on another surface 32B of board 32. Cover 34 covers component 33 and surface 32A having the component mounted thereon. Transmitter 501 is located in the tip of capsule 30. Helical antenna 31 is arranged to have distances L1, L2, and L3 which are longer than 2mm away from capsule 30. This arrangement reduces the change of load to antenna 31 caused by a change in external conditions around capsule 30, and improves the gain of antenna 31. The Improvement of the gain can reduce the size of antenna 31. Trimming part 61 shown in Fig. 5 is provided on surface 32B of board 32.

Fig. 3 is a perspective view of antenna 31. Helical antenna 31 includes core 41 made of magnetic material and electrically-conductive wire 40 wound around the core. This structure reduces a loss caused by a matching circuit more than an antenna having an air core, and allows helical antenna 31 to be mounted easily.

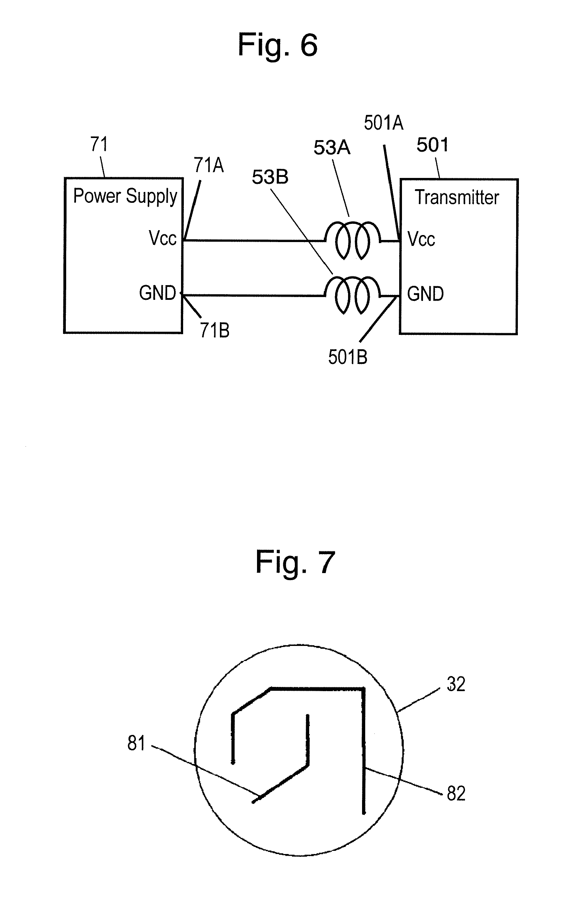

Fig. 6 is a block diagram of transmitter 501 and power supply 71. Power output 71A and ground 71B of power supply 71 are connected to power input 501A and ground 501B of transmitter 501 via inductors 53A and 53B, respectively. The inductance of each of inductors 53A and 53B is selected to exhibit a large impedance at the center frequency of the modulated wave emitted from antenna 16. According to Embodiment 1, the center frequency of the modulated wave is 315MHz. The inductance of each of inductors 53A and 53B is 100nH. Inductors 53A and 53B equivalently separate power output 71A and ground 71B of power supply 71 from power input 501A and ground 501B of transmitter 501 at the center frequency of the modulated wave, thereby reducing an influence of power output 71A and ground 71B to helical antenna 31, and increasing the gain of the antenna. Inductors 53A and 53B may be made of circuit patterns, and may be made of any form of a conductor exhibiting a large impedance at the center frequency of the modulated wave, having the same effects.

Fig. 7 illustrates a circuit pattern of transmitter 501. A ground line is made of a fine-line pattern similar to a signal line. This structure reduces an influence of the ground line to helical antenna 31, and increase the gain of the antenna.

Fig. 8A is a block diagram of another transmitter in accordance with Embodiment 1. Fig. 8B is a block diagram of still another transmitter in accordance with Embodiment 1. Transmitter shown in Fig. 8A includes buffer amplifier 91 connected between voltage-controlled oscillator 13 and antenna 16. Transmitter shown in Fig. 8B includes attenuator 92 connected between voltage-controlled oscillator 13 and antenna 16. Even when the impedance or gain of antenna 16 changes according to external influences to the antenna, an input impedance of buffer amplifier 91 or attenuator 92 does not change so much, thereby avoiding impedance mismatch at output of voltage-controlled oscillator 13.

Fig. 1B is a block diagram of receiver 601 of the transmission/reception system in accordance with Embodiment 1. Band-pass filter 201 removes unnecessary high-frequency waves in the modulated wave input into antenna 200. Then the wave is amplified by low-noise amplifier 202, and supplied to input port 203A of mixer 203. A local oscillation signal generated by frequency synthesizer 601A is supplied to another input port 203B of mixer 203. Frequency synthesizer 601A includes reference signal oscillator 207 for providing a fixed reference frequency generated by a quarts oscillator or other means, voltage-controlled oscillator 205, and phase-locked loop (PLL) circuit 204. Mixer 203 mixes the signals supplied to input ports 203A and 203B, and supplies an intermediate-frequency (IF) signal from output port 203C. The IF signal supplied from mixer 203 matches with center frequency Fc of a passing band of band-pass filter 211. Thus, band-pass filter 211 suppresses thermal noises of unnecessary frequencies around the frequency of the IF signal. Detector 209 demodulates the IF signal supplied from band-pass filter 211, and outputs voltages corresponding to data "0" and "1", respectively.

PLL circuit 204 has reference input port 204A for receiving a reference signal generated by reference signal oscillator 207, and comparison input port 204B for receiving a signal generated by voltage-controlled oscillator 205. PLL circuit 204 compares the phase of the reference signal generated by reference signal oscillator 207 with the phase of the signal generated by voltage-controlled oscillator 205, and outputs a control voltage which corresponds to the phase difference between these signals from output port 204C to frequency-control port 205A of voltage-controlled oscillator 205. Controller 210 supplies data to data input port 204D of PLL circuit 204 as to control the oscillation frequency of voltage-controlled oscillator 205. The IF signal supplied from mixer 203 is supplied to common port 212A of SPDT switch 212. SPDT switch 212 supplies the IF signal selectively to band-pass filter 211 and IF amplifier 208 via ports 212B and 212C, respectively. When common port 212A of switch 212 is connected to port 212B and disconnected from port 212C, IF amplifier 208 amplifies the IF signal supplied from mixer 203. Port 213C of SPDT switch 213 is connected to the output of IF amplifier 208. Port 212B of SPDT switch 213 is connected to reference signal oscillator 207. Common port 213A of SPDT switch 213 is connected to reference input port 204A of PLL circuit 204. Thus, switch 213 selectively supplies the IF signal supplied from IF amplifier 208 and a reference signal generated from reference signal generator 207 into reference input port 204 of PLL circuit 204.

Fig. 1C is a block diagram of PLL circuit 204. Frequency divider 1201 divides the frequency of the signal supplied to reference input port 204A by a dividing ratio of 1/R1. Frequency divider 1202 divides the frequency of the signal supplied to reference input port 204B by a dividing ratio of 1/R2. Phase comparator 1203 compares the phases of the signals supplied from frequency dividers 1201 and 1202, and outputs a signal corresponding to the phase difference between these signals. Charge pump 1204 and low-pass filter 1205 supply, from output port 204C, a voltage corresponding to the phase difference between the signals supplied to phase comparator 1203.

An operation of receiver 601 in accordance with Embodiment 1 will be described.

First, controller 210 connects common port 212A of SPDT switch 212 to port 212C, and disconnects common port 212A from port 212B. Controller 210 connects common port 213A of SPDT switch 213 to port 213C, and disconnects common port 213A from port 213B. The IF signal supplied from IF amplifier 208 is supplied to reference input port 204A of PLL circuit 204 via switches 211 and 213. A modulated signal of frequency F1 is received by antenna 200 and amplified by low-noise amplifier 202. Mixer 203 mixes the modulated signal with a local oscillation signal of frequency F2 supplied from voltage-controlled oscillator 205 of frequency synthesizer 601A, and converts these signals into an IF signal of frequency (F1-F2). Frequency (F1-F2) is center frequency Fc of band-pass filter 211, thus being a predetermined frequency. When frequency F1 of the modulated wave transmitted from transmitter 501 and received by antenna 200 varies from the reference frequency, oscillation frequency F2 of voltage-controlled oscillator 205 is necessarily changed accordingly. Frequency synthesizer 601A stabilizes these frequencies based on the following relation.

$$(F1\text{-}F2)/R1 = F2/R2 \ ... \ (\text{Equation 1})$$

When these frequencies are stable, A/D converter 214 converts the control voltage to be supplied to frequency control port 205A of voltage-controlled oscillator 205 into digital data. Controller 210 stores a reference control voltage to be supplied to frequency control port 205A of frequency-controlled oscillator 205 when the modulated wave of frequency F1 has a reference frequency and frequency (F1-F2) of the IF signal matches with frequency Fc. When antenna 200 receives a modulated wave deviating from the reference frequency, controller 210 detects, based on the data supplied from A/D converter 211, a frequency deviation of the reference local oscillation signal corresponding to the modulated wave of frequency F1 from the local oscillation signal corresponding to the modulated wave of the reference frequency. Controller 210 inputs control data into data input port 204D of PLL circuit 204, and determines dividing ratios R1 and R2 of frequency dividers 1201 and 1202. Specifically, controller 211 supplies control data to control data input port 204D to determine dividing ratios R1 and R2 so that intermediate frequency (F1-F2) obtained from the modulated wave of frequency F1 matches with center frequency Fc of band-pass filter 211, that is, voltage-controlled oscillator 205 generates the local oscillation signal of frequency F2 obtained by the following equation derived from Equation 1.

$$F2 = Fc \cdot R2/R1 \ ... \ (\text{Equation 2})$$

Controller 210 may store a table that indicates the control voltages that are supplied to voltage-controlled oscillator 205 and converted into digital data by A/D converter 211, and dividing ratios R1 and R2 corresponding to the control voltages. Controller 210 supplies control data to PLL circuit 204 in order to determine dividing ratios R1 and R2 based on the table in.

Alternatively, controller 210 may store an approximate formula indicating the relation between the control voltage supplied to voltage-controlled oscillator 205 and oscillation frequency F2 of voltage-controlled oscillator 205. Controller 210 derives necessary frequency F2 from the approximate formula, determines dividing ratios R1 and R2 based on Equation 2, and supplies the control data to PLL circuit 204.

After determining oscillation frequency F2 and dividing ratios R1 and R2 as described above, controller 210 connects common port 212A of switch 212 to port 212B and disconnects common port 212A from port 212C, and connects common port 213A of switch 213 to port 213B and disconnects common port 213A from port 213C. Then, receiver 601 operates ordinarily. When the frequency of the reference signal generated by reference signal oscillator 207 is equal to center frequency Fc of band-pass filter 211, mixer 203 mixes the local oscillation signal generated by voltage-controlled oscillator 205 via PLL circuit 204 at determined dividing ratios R1 and R2 with the signal from low-noise amplifier 202, and outputs an IF signal of frequency Fc. When the frequency of the reference signal generated by reference signal oscillator 207 is 1/R3 of center frequency Fc of band-pass filter 211, controller 210 determines the dividing ratios of frequency dividers 1201 and 1202 to R1 and R2/R3, respectively.

Frequency divider 1201 for dividing the frequency of the signals supplied to reference input port 204A may be eliminated. In this case, ratio R1 is equal to 1.

Switch 212 may be eliminated. In this case, the output of voltage-controlled oscillator 205 is always supplied to the IF amplifier and band-pass filter 211.

Controller 210 may preferably determine the oscillation frequency of voltage-controlled oscillator 205, i.e. dividing ratios R1 and R2, by the above procedure intermittently at intervals corresponding to environment and applications.

Transmitter 501 shown in Fig. 1A of Embodiment 1 has a structure simpler than a conventional transmitter under feedback control, thus having a smaller size and consuming smaller power. Receiver 601 works effective even when the modulated wave transmitted by transmitter 501 has an unstable frequency.

EXEMPLARY EMBODIMENT 2

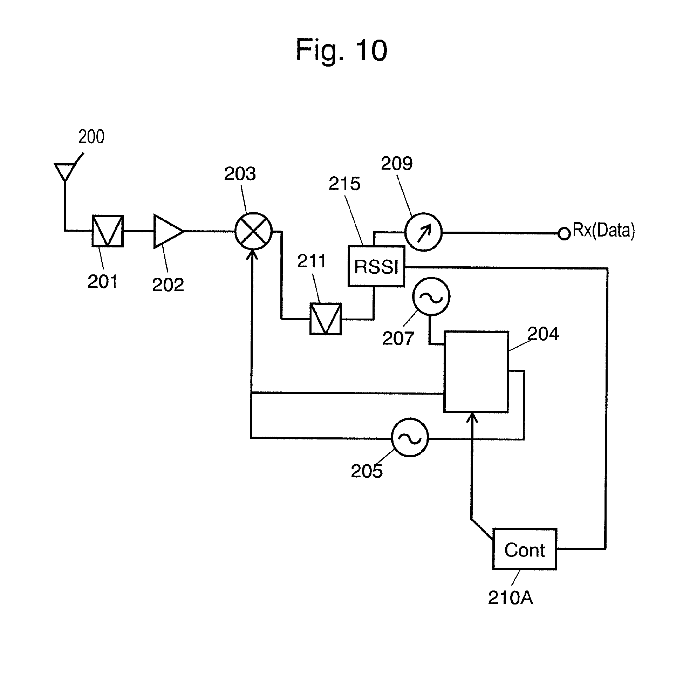

Fig. 10 is a block diagram of receiver 602 in accordance with Exemplary Embodiment 2 of the present invention. The same components of receiver 601 of Embodiment 1 shown in Fig. 1B are denoted by the same reference numerals, and their descriptions will be omitted.

Receiver 602 includes RSSI detector 215 that detects electric field strength of a signal supplied from band-pass filter 211. A wave received from the antenna is converted into an intermediate-frequency (IF) signal by mixer 203, and supplied to RSSI detector 215. RSSI detector 215 detects the electric field strength of the IF signal and inputs the detected strength to controller 210A. Controller 204A changes control data supplied to phase-locked loop (PLL) circuit 204 as to change an oscillation frequency of voltage-controlled oscillator 205, and determines the data, so that the electric field strength detected by RSSI detector 215 becomes the largest. Voltage-controlled oscillator 205 generates a signal of a frequency corresponding to the determined data. Receiver 602 performs ordinal receiving operation.

EXEMPLARY EMBODIMENT 3

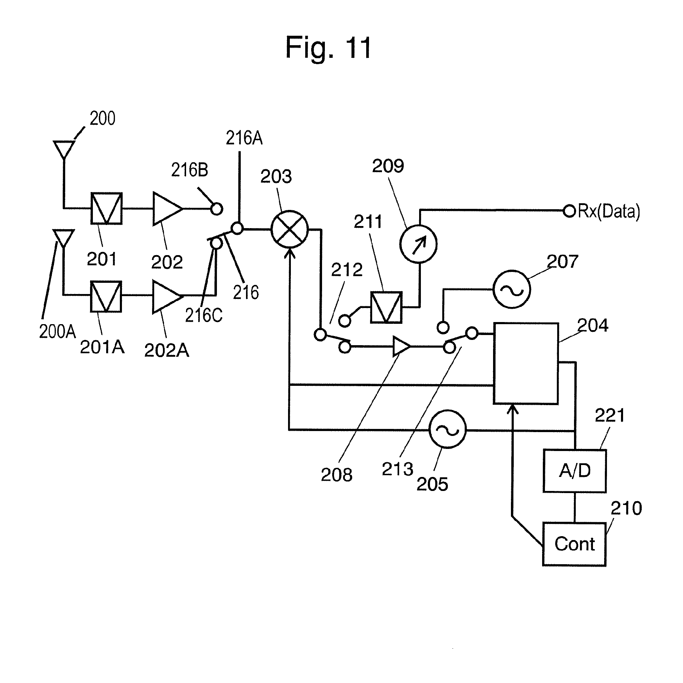

Fig. 11 is a block diagram of receiver 603 in accordance with Exemplary Embodiment 3 of the present invention. The same components of receiver 601 of Embodiment shown in Fig. 1B are denoted by the same reference numerals, and their description will be omitted.

Receiver 603 includes antenna 200, band-pass filter 201, and low-noise amplifier 202 which are provided for signals of a first frequency band. Receiver 603 further includes antenna 200A, band-pass filter 201A, and low-noise amplifier 202A which are provided for signals in a second frequency band different from the first frequency band. Common port 216A of switch 216 is connected to port 216B for receiving a signal in the first frequency band. Common port 216A of switch 216 is connected to port 216C for receiving a signal in the second frequency band. Controller 210A determines control data to allow voltage-controlled oscillator 205 to generate a signal of a frequency so that mixer 203 supplies IF signals of frequencies identical to each other for the signals in the first and second frequency bands different from each other. The frequency of the local oscillation signals is determined to causing so that the intermediate frequency is constant even when signals of frequencies different from each other are received. Hence, circuits after mixer 203 can be commonly work for the signals in two different frequency bands. This structure allows receiver 603 to be compatible with systems having the same detection methods and different frequency bands. Further, when receiving interference waves, receiver 603 can select the best frequency band among the frequency bands. Each of the number of antennas and the number of low-noise amplifiers may be more than two.

INDUSTRIAL APPLICABILITY

A transmitter of a transmission/reception system according to the present invention has a small size and consumes small power. A receiver has a simple structure and high sensitivity. The receiver is effective when a transmission frequency of the transmitter is unstable.

**Claims**

1. A transmission/reception system adaptable to be used for an encapsulated endoscope that can be swallowed by a living organism, said system comprising:

a transmitter including

a voltage converter for generating a voltage according to transmission data, a voltage-controlled oscillator for generating a signal of a frequency corresponding to the voltage generated by the voltage converter under non-feedback control, and a first antenna for emitting the signal generated by the voltage-controlled oscillator;

a receiver including

a second antenna for receiving the signal emitted from the first antenna, a first amplifier for amplifying the signal received by the second antenna, an oscillator for generating a local oscillation signal, a mixer for mixing the signal amplified by the first amplifier with the local oscillation signal and converting the signal amplified by the first amplifier into an intermediate-frequency (IF) signal, a detector for detecting the IF signal, and a controller for changing a frequency of the local oscillation signal according to the frequency of the received signal, so that the IF signal has a predetermined frequency.

2. A transmitter used in a transmission/reception system adaptable to be used for an encapsulated endoscope that can be swallowed by a living organism, said transmitter comprising:

a voltage converter for generating a voltage according to transmission data; a voltage-controlled oscillator for generating a signal of a frequency corresponding to the voltage generated by the voltage converter under non-feedback control; and an antenna for emitting a signal generated by the voltage-controlled oscillator.

3. The transmitter of claim 2, wherein the voltage-controlled oscillator includes a resonator, a variable capacitor, a transistor, an inductor, a capacitor, and a resistor.

4. The transmitter of claim 3, wherein the resonator includes a chip inductor and a trimming part connected to the chip inductor.

5. The transmitter of claim 4, further comprising a board having the chip inductor mounted thereon, wherein the trimming part is made of a circuit pattern provided on the board.

6. The transmitter of claim 3, wherein the resonator comprises a surface acoustic wave resonator.

7. The transmitter of claim 2, further comprising:

a power supply for driving the voltage converter and the voltage-controlled oscillator, wherein the power supply has a power-supply ground, and the voltage converter and the voltage-controlled oscillator have a ground; and an inductor connected between the ground and the power-supply ground.

8. The transmitter of claim 7, further including a circuit pattern functioning as the inductor.

9. The transmitter of claim 2, wherein the voltage converter includes:

a first resistor having a first end, and a second end connected to an output of the transmission data generator; a second resistor having a first end and a second end connected to the first end of the first resistor, the second resistor voltage-dividing the output of the transmission data generator; and a capacitor connected between the first end of the first resistor and the second end of the first resistor.

10. The transmitter of claim 2, further comprising a board, the board having a first surface and a second surface opposite to the first surface, the first surface having the voltage-controlled oscillator mounted thereon, the second surface having the antenna mounted thereon.

11. The transmitter of claim 2, wherein the antenna comprises a helical antenna, the helical antenna including a core and a wire wound around the core.

12. The transmitter of claim 11, wherein the core of the antenna comprises magnetic material.

13. The transmitter of claim 2, further comprising a capsule for accommodating the transmission data generator, the voltage converter, the voltage-controlled oscillator, and the antenna therein.

14. The transmitter of claim 13, wherein the antenna is arranged away from the capsule by a distance longer than 2.0mm.

15. The transmitter of claim 14, wherein the antenna comprises a helical antenna including a core and a wire wound around the core.

**16.** The transmitter of claim 15, wherein
the capsule has an axis in a longitudinal direction, and
the wire of the antenna is wound like a coil having an axis in parallel with the axis of the capsule.

**17.** The transmitter of claim 15, wherein the core of the antenna comprises magnetic material.

**18.** The transmitter of claim 2, further including a fine-line circuit pattern functioning as a ground.

**19.** The transmitter of claim 2, further comprising a buffer amplifier coupled between the voltage-controlled oscillator and the antenna.

**20.** The transmitter of claim 2, further comprising an attenuator coupled between the voltage-controlled oscillator and the antenna.

**21.** A receiver comprising:

a first antenna;
a first amplifier for amplifying a signal received by the first antenna;
an oscillator for generating a local oscillation signal;
a mixer for mixing the signal amplified by the first amplifier with the local oscillation signal and converting the signal amplified by the first amplifier into an intermediate-frequency (IF) signal;
a detector for detecting the IF signal; and
a controller for changing the frequency of the local oscillation signal according to the frequency of the received signal, so that the IF signal has a predetermined frequency.

**22.** The receiver of claim 21, wherein the oscillator comprises a second voltage-controlled oscillator received a control voltage, and the local oscillation signal has a frequency corresponding to the received control voltage, said receiver further comprising:

a reference signal oscillator;
a first switch for outputting selectively one of a signal supplied from the reference signal oscillator and the IF signal;
a first frequency divider for dividing a frequency of the signal supplied from the first switch;
a second frequency divider for dividing a frequency of the local oscillation signal generated by the second voltage-controlled oscillator;
a phase comparator for comparing a phase of a signal supplied from the first frequency divider with a phase a signal supplied from the second frequency divider; and
a charge pump for inputting, into the second voltage-controlled oscillator as the control voltage, a voltage corresponding to a phase difference between the signal supplied from the first frequency divider and the signal supplied from the second frequency divider.

**23.** The receiver of claim 22, wherein the controller determines a first dividing ratio of the first frequency divider and a second dividing ratio of the second frequency divider according to the control voltage.

**24.** The receiver of claim 23, wherein
the controller is operable to determine the dividing ratio of the first frequency divider and the dividing ratio of the second frequency divider according to the control voltage when the first switch outputs the reference IF signal;
when the first switch outputs the signal supplied from the reference signal oscillator, the first frequency divider divides the frequency of the signal supplied from the reference signal oscillator by the determined first dividing ratio; and
when the first switch outputs the signal supplied from the reference signal oscillator, the second frequency divider divides the frequency of the local oscillation signal by the determined second dividing ratio,.

**25.** The receiver of claim 23, wherein the controller has a table indicating the first dividing ratio and the second dividing ratio corresponding to a value of the control voltage.

**26.** The receiver of claim 22, further comprising:

a second antenna for receiving a signal of a frequency different from a frequency of the signal received by the first antenna;
a second amplifier for amplifying the signal received by the second antenna; and
a second switch for selectively outputting one of the signal amplified by the first antenna and the signal amplified by the second amplifier.

**27.** The receiver of claim 21, further comprising an RSSI detector for detecting electric field strength of the IF signal, wherein the controller is operable to determine the first dividing ratio and the second dividing ratio, so that the detected electric field strength becomes largest.

# Fig. 1A

# Fig. 1B

# Fig. 1C

# Fig. 2

# Fig. 3

# Fig. 4

# Fig. 5

# Fig. 6

71

71A

53B

53A

501A

501

Power Supply

Transmitter

Vcc

Vcc

GND

GND

71B

501B

# Fig. 7

32

81

82

# Fig. 8A

13

Voltage-Controlled Oscillator

91

AMP

16

Antenna

# Fig. 8B

13

Voltage-Controlled Oscillator

92

ATT

16

Antenna

# Fig. 9

Data

12

Voltage Converter

13

Voltage-Controlled Oscillator

SAW Resonator

16

Antenna

14

21

# Fig. 10

# Fig. 11

# Fig. 12

# Fig. 13A

```
        318              317                      316
   ┌────────┐      ┌──────────────┐          ┌─────────┐
   │ Voltage │────▶│Voltage-Controlled│─────●────▶│ Antenna │
Data│  Adder  │    │   Oscillator  │     │    │         │
   └────────┘      └──────────────┘     │    └─────────┘
        ▲                               │
        │                               ▼
   ┌ ─ ─│─ ─ ─ ─ ─ ─ ─ ─ ─ ─ ─ ─ ─ ─ ─ ─ ─ ─ ─ ─ ─ ─ ┐
        │                    311 ┌──────────────┐
   │┌────┴───┐                   │  Frequency   │          │
    │  LPF   │◀──────        315 │   Divider    │
   │└────────┘                   └──────┬───────┘          │
        ▲                               │
   │    │                               ▼                  │
   ┌────┴───┐              ┌──────────────┐   ┌──────────┐
   ││ Charge │◀─────────────│    Phase     │◀──│Oscillator││
    │  Pump  │              │  Comparator  │   │          │
   │└────────┘              └──────────────┘   └──────────┘│
     314                           313            312
   └ ─ ─ ─ ─ ─ ─ ─ ─ ─ ─ ─ ─ ─ ─ ─ ─ ─ ─ ─ ─ ─ ─ ─ ─ ┘
     310
```

# Fig. 13B

<div style="text-align:center">**INTERNATIONAL SEARCH REPORT**</div>

| | International application No. |
|---|---|
| | PCT/JP2005/009420 |

**A. CLASSIFICATION OF SUBJECT MATTER**
Int.Cl$^7$ A61B1/00

According to International Patent Classification (IPC) or to both national classification and IPC

**B. FIELDS SEARCHED**

Minimum documentation searched (classification system followed by classification symbols)
Int.Cl$^7$ A61B1/00-1/32

Documentation searched other than minimum documentation to the extent that such documents are included in the fields searched
Jitsuyo Shinan Koho        1922-1996   Jitsuyo Shinan Toroku Koho   1996-2005
Kokai Jitsuyo Shinan Koho  1971-2005   Toroku Jitsuyo Shinan Koho   1994-2005

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| Y | JP 2001-245844 A (Asahi Optical Co., Ltd.), 11 September, 2001 (11.09.01), Par. Nos. [0012] to [0014]; Fig. 2 (Family: none) | 1-27 |
| Y | JP 5-315896 A (Sony Corp.), 26 November, 1993 (26.11.93), Par. Nos. [0020], [0021]; Fig. 1 (Family: none) | 1-27 |

☐ Further documents are listed in the continuation of Box C.    ☐ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 17 August, 2005 (17.08.05) | 06 September, 2005 (06.09.05) |

| Name and mailing address of the ISA/ | Authorized officer |
|---|---|
| Japanese Patent Office | |
| Facsimile No. | Telephone No. |

Form PCT/ISA/210 (second sheet) (January 2004)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 0165995 A